# EUROPEAN PATENT SPECIFICATION

(11) **EP 0 289 550 B1**
(45) Date of publication and mention of the grant of the patent: **10.04.1996**
(21) Application number: 87907206.4
(22) Date of filing: 20.10.1987
(51) Int. Cl.: A61K 39/245, A61K 39/12

(54) **VACCINE FOR USE IN THE THERAPEUTIC TREATMENT OF HSV**
IMPFSTOFF ZUR BEHANDLUNG VON HSV
VACCIN UTILISE POUR LE TRAITEMENT THERAPEUTIQUE DE HSV

(30) Priority: 20.10.1986 US 921213; 20.07.1987 US 79605
(43) Date of publication of application: 09.11.1988
(73) Proprietor: CHIRON CORPORATION, Emeryville, California 94608 (US)
(72) Inventor: BURKE, Rae, Lyn, San Francisco, CA 94117 (US); PACHL, Carol, Oakland, CA 94611 (US); VALENZUELA, Pablo, D., T., San Francisco, CA 94117 (US)
(74) Representative: Hallybone, Huw George
(86) International application number: US8702709
(87) International publication number: WO8802634

(56) References cited:
- WO-A-85/04587
- US-A- 4 642 333
- US-A- 4 661 349
- Science, Volume 227, No. 4693, issued March 1985 (USA), BERMAN, "Protection from Genital Herpes Simplex Virus Type 2 Infection by Vaccination with Cloned. Type 1 Glycoprotein D". see pages 1490-1492.
- J Infect Dis, V157 no.5, pp897-902

## Description

### BACKGROUND OF THE INVENTION

### Field of the Invention

The herpes viruses include the herpes simplex viruses, comprising two closely related variants designated types 1 (HSV-1) and 2 (HSV-2). These types cross react strongly but can be distinguished by neutralization titrations. HSV-1 and HSV-2 are responsible for a variety of human diseases, such as skin infections, genital herpes, viral encephalitis and the like.

The herpes simplex virus is a double stranded DNA virus having a genome of about 150 to 160kbp packaged within an icosahedral nucleocapsid enveloped in a membrane. The membrane includes a number of virus-specific glycoproteins, the most abundant of which are gB, gC, gD and gE, where gB and gD are cross-reactive between types 1 and 2.

It is a matter of great medical and scientific interest to provide safe and effective vaccines for humans against both HSV-1 and HSV-2 and, where infection has occurred, therapies for treatment of the disease.

One promising approach has been the prophylactic use of isolated glycoproteins, which have been shown to provide protection when injected into mice subsequently challenged with live virus. However, the availability of the Herpes Simplex glycoproteins has heretofore been primarily dependent upon the growth of the virus and the isolation of the membranous proteins. The problems of commercial production of the glycoproteins associated with the handling of a dangerous pathogen, the maintenance of the virus in cell culture, the isolation of the glycoproteins free of the viral genome or portions thereof, have substantially precluded the use of the glycoproteins as vaccines. It would therefore be desirable to provide vaccines employing glycoproteins produced by methods other than by growth of the virus and isolation of the membranes proteins.

There is also substantial interest in developing methods for therapeutically treating herpes infections, i.e., treatment after the individual has been infected with the virus, which lessens or prevents recurrence of the disease. Since viral infections are normally resistant to treatment with antibiotics, other techniques which do not have significant side effects are of great interest. Although some drugs have been useful in the treatment of recurrent herpetic disease, in particular, Acyclovir, the need for continuous long term administration to suppress recurrent episodes may not be desirable, and drug resistant mutants arise during the course of treatment.

### DESCRIPTION OF THE RELEVANT LITERATURE

Eberle and Mou, J. of Infectious Diseases (1983) 148:436-444, report the relative titers of antibodies to individual polypeptide antigens of HSV-1 in human sera. Marsden et al., J. of Virology (1978) 28:624-642, report the location of a gene for a 117 kilodalton (kd) glycoprotein to lie within 0.35-0.40 map units on the genetic map of HSV by intertypic recombination between HSV-1 and HSV-2. Ruyechan et al., ibid. (1979) 29:677-697, also report the mapping of glycoprotein B gene to lie between 0.30-0.42 map units. Skare and Summers, Virology (1977) 76:581-595, report endonuclease cleavage sites for EcoRI, XbaI and HindIII on HSV-1 DNA. Roizman, Ann. Rev. Genetics (1979) 13:25-57, reports the organization of the HSV genomes. DeLucca et al., Virology (1982) 122:411, map several phenotypic mutants thought to lie in the gB1 structural gene between 0.345 to 0.368 map units.

Subunit vaccines extracted from chick embryo cells infected with HSV-1 or HSV-2 are described in U.S. Patents Nos. 4,317,811 and 4,374,127. See also, Hilfenhaus et al., Develop. Biol. Standard (1982) 52:321-331, where the preparation of a subunit vaccine from a particular HSV-1 strain (BW3) is described. Roizman et al., ibid, (1982) 52:287-304, describe the preparation of nonvirulent HSV-1 x HSV-2 recombinants and deletion mutants which are shown to be effective in immunizing mice. Watson et al., Science (1982) 218:381-384, describe the cloning and low level expression of the HSV-1 gD gene in E. coli, as well as expression of a cloned fragment by injection into the nuclei of frog oocytes. They also present the nucleotide sequence for the gD gene. Weis et al., Nature (1983) 302:72-74, report higher level expression of gD in E. coli. This polypeptide elicits neutralizinp antibodies in rabbits. Berman et al., Science (1983) 222: 524-527, report the expression of glycoprotein D in mammalian cell culture. Lasky et al., Biotechnology (June 1984) 527-532, report the use of this glycoprotein D for the immunization of mice. Cohen et al., J. Virol. (1984) 49:102-108, report the localization and chemical synthesis of a particular antigenic determinant of gD.

"Therapeutic" use of preparations of membrane proteins from HSV-infected cells for post-infection vaccine in humans are reported by Dundarov, S. et al., Dev. Biol. Standard (1982) 52:351-357; and Skinner, G. R. B. et al., ibid. (1982) 52:333-34.

### SUMMARY OF THE INVENTION

The present invention provides the use of
(a) an immunogenically active recombinant herpes simplex virus glycoprotein B (gB) polypeptide; and/or
(b) an immunogenically active recombinant herpes simplex virus glycoprotein D (gD) polypeptide;
in making a vaccine for the post-infection treatment of Herpes Simplex Virus (HSV).

The vaccine may be employed for therapeutic use against Herpes Simplex Virus Types 1 and 2. These therapeutics employ a combination of virus specific polypeptides produced by recombinant DNA technology. Particularly, HSV gB and gD may be produced by recombinant DNA technology in modified mammalian hosts and employed in combination. They may be used for the treatment of herpes simplex viral infections in animals, including humans. Said polypeptides are present in an amount effective to prevent recurrent HSV induced disease in an individual who is infected with HSV, when the vaccine is administered to the individual subsequent to primary infection with HSV.

### BRIEF DESCRIPTION OF THE DRAWINGS

Fig. 1 shows physical maps of HSV-1 and HSV-2, an EcoRI cleavage map for the prototype isomer arrangement, and a HindIII restriction map of HSV-2.

Fig. 2 shows a restriction map of the region of HSV-1 map which encodes gB1.

Fig. 3 is a restriction map of gB1 coding region.

Fig. 4 shows the DNA amino acid sequences of gB1 and gB2.

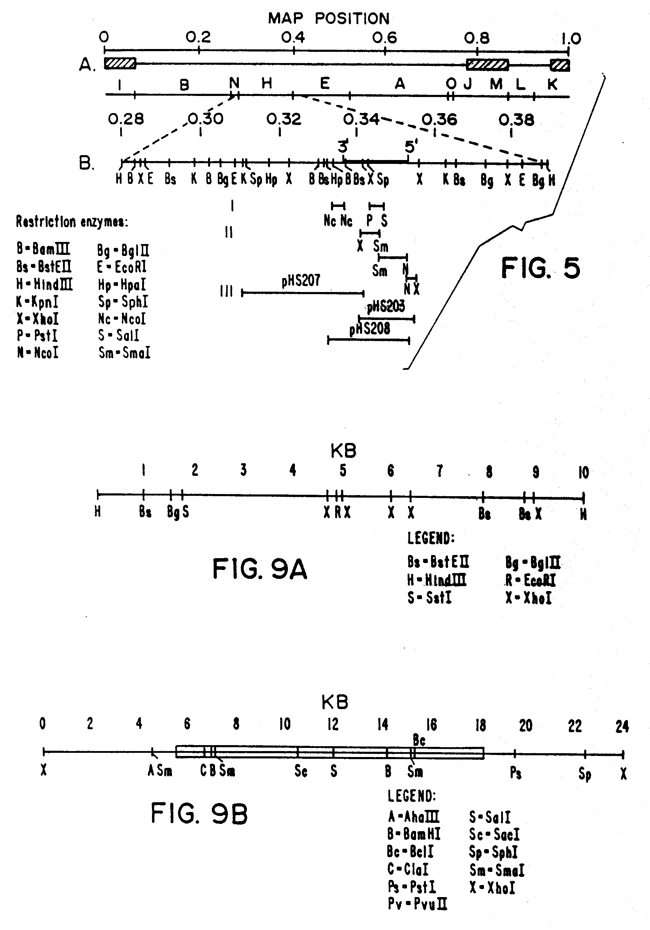

Fig. 5 is a physical map of HSV-2, indicating coding regions for gB2.

Fig. 6 is a map showing some significant features of the plasmid pHS137.

Fig. 7 is a restriction map of gB2.

Fig. 8 is a flow chart for the construction of pHS132, a mammalian expression vector for gD1.

Fig. 9 (A&B) is a physical map of HSV-2 indicating the coding region for gD2.

Fig. 10 is a flow chart of the construction of mammalian vectors for gD2.

Fig. 11 shows the effect of vaccination with recombinant gB-gD after primary infection on recurrent herpetic disease.

Fig. 12A shows the effect of immunization with herpes virus glycoproteins on the rate of recurrent herpetic infections.

Fig. 12B shows the difference in weekly recurrence rates between control and immunized guinea pigs.

Fig. 13 is a graph showing the effect of the time of administration of gBgD vaccine on the recurrence of herpetic disease.

### DESCRIPTION OF THE SPECIFIC EMBODIMENTS

### Vaccines

The vaccines of the invention employ recombinant HSV glycoproteins B and D of both types 1 and 2. Mature (full length) gB and gD proteins may be used as well as fragments, precursors and analogs that are immunologically equivalent (i.e., provide protection against infection) to the mature proteins. As used in the claims, the terms "glycoprotein B polypeptide" and "glycoprotein D polypeptide" are intended to include such fragments, precursors, and analogs. The recombinant gB and gD polypeptides are produced in eukaryotic cells preferably yeast or mammalian cells, most preferably mammalian cells. Fragments will be at least about 15 amino acids and preferably at least about 30 amino acids in length. The vaccines may comprise a mixture of type 1 polypeptides, a mixture of type 2 polypeptides or a mixture of both type 1 and type 2 polypeptides, or any of the individual polypeptides.

The mixtures of gB and gD polypeptides may be used neat, but normally will be used in conjunction with a physiologically and pharmacologically acceptable medium, generally water, saline, phosphate buffered saline, sugar, etc., and may be employed with a physiologically acceptable adjuvant, e.g., aluminum hydroxide, muramyl dipeptide derivatives and the like. As shown in Example 6.3, a variety of adjuvants may be efficacious. The choice of an adjuvant will depend at least in part on the stability of the vaccine containing the adjuvant, the route of administration, the efficacy of the adjuvant for the species of the individual being vaccinated, and, in humans whether or not the adjuvant has been approved for human use by the Food and Drug Administration. The vaccine may be delivered in liposomes and/or in conjunction with immunomodulators such as interleukins 1 and 2. The vaccines may be administered by any convenient parenteral route, e.g., intravenously, intraarterially, subcutaneously, intradermally, intramuscularly or intraperitoneally. It may be advantageous to administer split doses of vaccines which may be administered by the same or different routes. The vaccines are administered subsequent to an initial infection with herpes simplex virus.

The efficacy of post-infection administration is demonstrated in Example 6.1. The effect of the vaccine on increasing the host immune response even after viral infection is shown in Example 6.2. The surface viral glycoproteins of herpes simplex virus have been shown to be antigens recognized by antibodies mediating viral neutralization and antibody-dependent cell-mediated cytotoxicity (ADCC). Norrild, B., et al, Infect. Immun. (1980) 28:38-44. Yet patients with frequently recurring HSV infections often have high levels of both neutralizing antibodies and ADCC. Corey, L. and Spear, P.G., Eng. N., J. Med. 314:686-691, 1986. For such patients, the predominant elicitors of HSV specific antibody has been shown to be the viral glycoproteins. Eberle, R., Mou, S.W. and Zaia, J.A., J. Gen. Virol. 65:1839-1843, 1984. Therefore, the therapeutic utility for the treatment of recurrent genital herpes of a recombinant subunit vaccine composed of only two viral glycoproteins and no additional viral antigens was unexpected.

Glycoproteins B and D may be used without modification. However, when smaller related polypeptides are used, such as fragments or the like, and their molecular weight is less than about 5000 daltons, e.g., 1500 to 5000 daltons, modification may be required to elicit the desired immune response. The smaller haptens should be conjugated to an appropriate immunogenic carrier such as tetanus toxoid or the like.

It is also possible to link short DNA fragments encoding the gB or gD polypeptides to genes expressing proteins from other pathogenic organisms or viruses. In this way, the resulting fused proteins may provide immunity for more than one disease.

The total amount of recombinant gB and gD polypeptides employed per dose will usually be about 0.1µg to 2mg/kg, more usually about 0.5µg to 1mg/kg and particularly about 0.5 to 10µg/kg of host body weight. The ratio of gB to gD in the vaccine will usually be about 0.1:1 to 10:1, more usually about 0.5:1 to 10:1 and preferably about 0.5:1 to 5:1. The dose may be administered repeatedly at daily to weekly intervals, and usually two to four week intervals, usually not more than about two to ten times. However, as shown in Example 6.4, the time of administration after primary exposure to the virus affects the rate of recurrent disease. Therefore, it will probably be necessary to determine the most efficacious time(s) of administration depending upon the species and/or individuals to be treated. The most efficacious times can be determined by routine testing using, for example, disease symptomotology or antibody titer to monitor the disease state. In addition, the data in Example 6.4 demonstrates that administration of the vaccine during an acute phase of the disease, i.e., when the individual manifests HSV-induced lesions on the body, significantly lessens the recurrence of the disease.

### Recombinant Glycoprotein B

The preparation of recombinant gB polypeptides is described in detail in International Application No. PCT/US85/00587, which is International Publication No. WO 85/04587, published 24 October 1985. A brief description of the materials and methods used to make recombinant gB polypeptides follows.

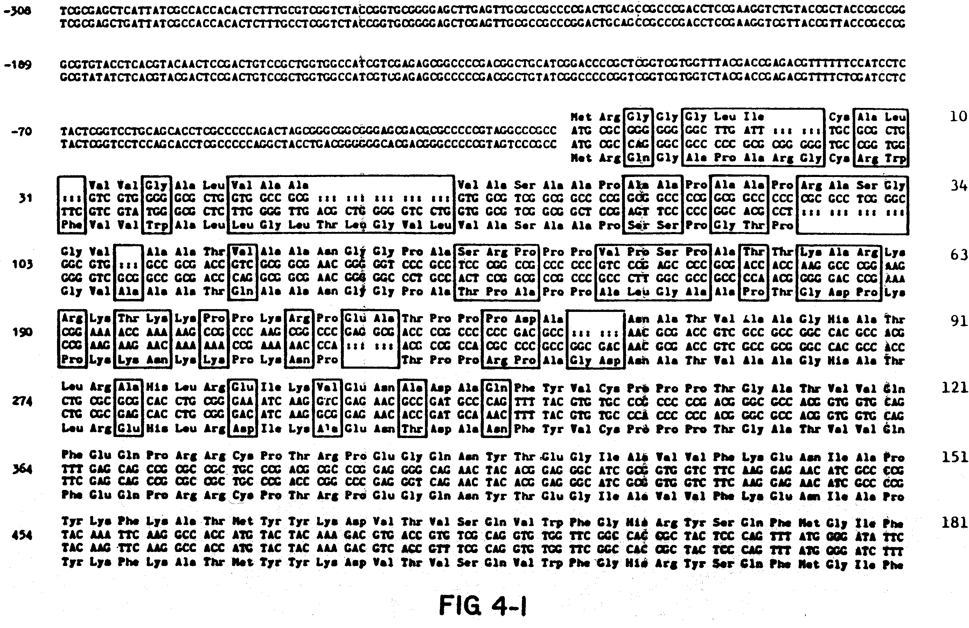

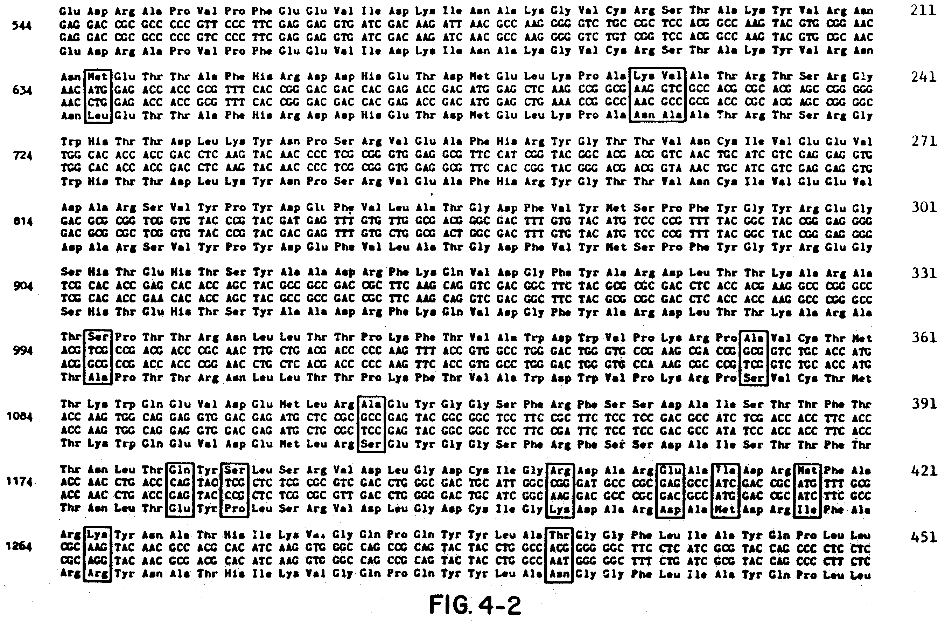

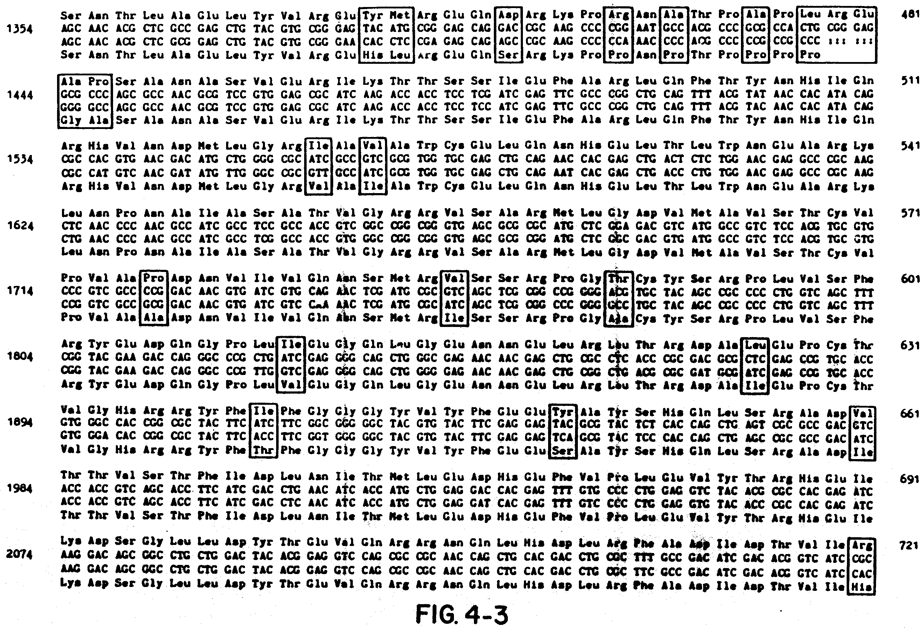

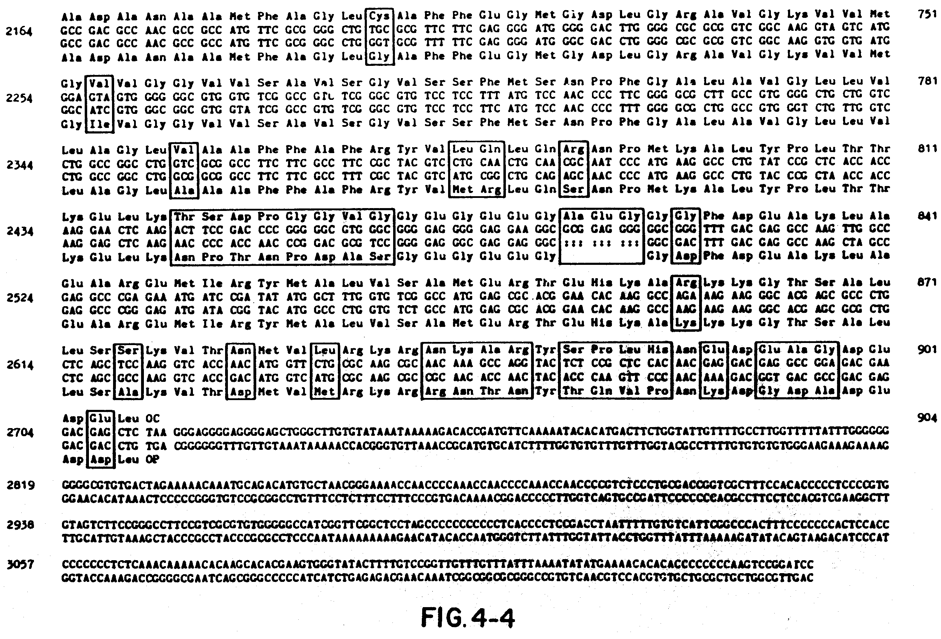

Figure 4 in the Experimental section provides the nucleotide sequence for gB1 strain Patton, as well as the amino acid sequence coded by the nucleotide sequence. Figure 4 also shows the substantial homology between gB1 and gB2. The nucleotide sequence may be varied in numerous ways. Various fragments may be employed having independent functions, which may be joined to proteins other than the mature gB. In addition, the various codons may be modified so as the encode for the same amino acids, but provide more efficient expression in accordance with the nature of the host. For example, the codons may be modifed in accordance with the frequency of occurrence of a particular codon in one or more proteins or groups of proteins, e.g., glycolytic proteins, which contribute to a high proportion of the total proteins of a particular host, e.g., yeast. In some instances one or more codons may be modified to code for a different amino acid, substituting one amino acid for another amino acid, where the effect of the change is not detrimental to the immunogenicity of the protein or to other biological factors of interest. It may be desirable in some instances to add amino acids to the N-terminus or C-terminus, where such additional amino acids may provide for a desired result. This can be readily achieved by providing for additional codons at the 5'-or 3'-termini of the sequence encoding the mature gB1 or its precursor. In addition, while the amino acid sequence of gB2 may differ from that of gB1 by as much as 20 number percent, other strains of HSV-1 or of HSV-2 will have gB glycoproteins the same as or similar to gB1 strain Patton or gB2 strain 333, respectively, usually differing by fewer than 5 number percent, more usually differing by fewer than 2 number percent, and frequently differing by fewer than 0.5 number percent amino acids from the amino acid sequence of gB1 strain Patton or gB2 strain 333.

The gB1 sequence, particularly gB1 strain Patton, may be divided into four domains beginning at the N-terminus of the protein: first hydrophobic region extending from amino acid 1 to about amino acid 30; a region of variable polarity extending from the first hydrophobic region to about amino acid 726; a second hydrophobic region extending from said variable polarity region to about amino acid 795, and a second variable polarity region extending to the C-terminus at amino acid 904.

Since gB is a membrane glycoprotein, based on analogy with other glycoproteins, the first hydrophobic region may be considered the signal leader sequence directing secretion and/or membrane location. The first sequence of variable polarity would then be external to the membrane and serve as the recognition sequence, to the extent that gB serves as a receptor for another protein or as an immunogen in a vaccine. The second hydrophobic sequence may serve as a transmembrane integrator sequence (often termed the "anchor"). The second variable polarity amino acid sequence would be expected to be in the cytoplasm and, to the extent that a receptor is external to the transmembrane integrator sequence, may serve to modulate one or more cytoplasmic processes.

The polynucleotide sequence encoding for the precursor to gB or functional fragments thereof may be cloned and expressed by inserting the polynucleotide sequence into an appropriate expression vector and introducing the resulting expression product construct into a compatible host. The coding fragments will be less than about 0.1 map unit, usually less than about 0.05 map unit where 1.0, map unit is the size of the entire HSV genome. The expression vector may be a low or high multicopy vector which exists extrachromosomally or integrated into the genome of the host cell and may provide for secretion or excretion of the polypeptide of interest or retention of the polypeptide of interest in the cytoplasm or in the membrane. A large number of expression vectors have been published in the literature and are generally available for use in eukaryotic hosts, including yeast, e.g., S. cerevisiae, and a wide variety of immortalized mammalian cells, such as mouse cells, monkey cells, hamster cells, e.g., 3T3, Vero, Chinese Hamster Ovary cells (CHO), etc or primary cell lines. Depending upon the host, where secretion is desired, either the natural or unnatural secretory leader sequence may be employed. The processing signals for cleavage of the secretory leader may be the natural signals or the signals associated with the unnatural secretory leader or both in tandem.

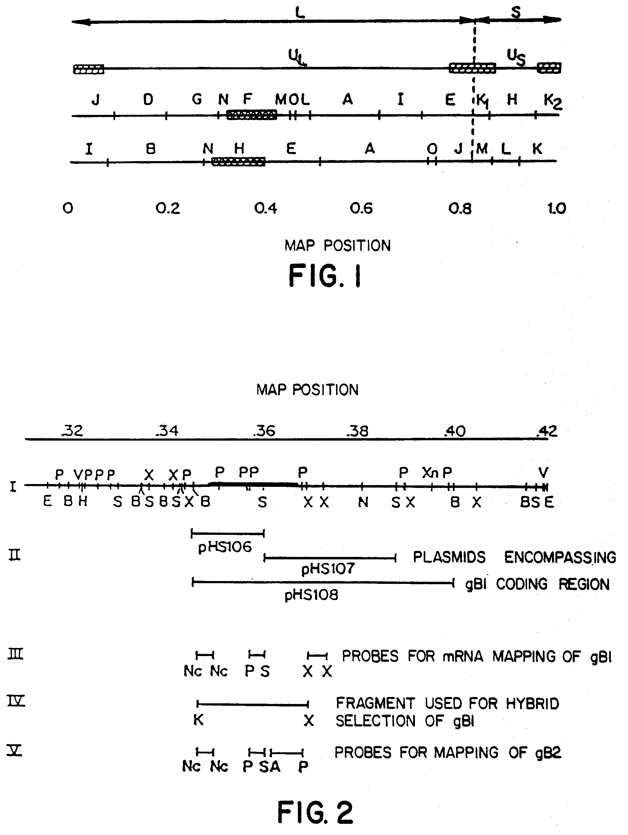

In order to obtain the polynucleotide sequence encoding for gB1-Patton, the location of the gB1 coding sequences on the EcoRI restriction fragment F was mapped. Three subfragments of the F fragment were isolated and subcloned into pBR322 (Fig. 2). DNA fragments from these subclones were then used to probe Northern blots of Poly A⁺ mRNA isolated from HSV-1 infected cells. Fragments which hybridized to mRNA of the size expected for gB were presumed to lie within the gB coding region. The direction of transcription of gB was also elicited by determining which strand of the DNA probes hybridized with the mRNA. To verify the identity of the gB sequence, DNA fragments were used to hybrid-select HSV-1 mRNA, which was then translated in vitro and the resulting proteins analyzed for gB using a gB specific antibody.

The gB1 coding fragment may now be manipulated in a variety of ways, including restriction mapping and sequencing, so as to establish the restriction sites and the open reading frame regions for expression. The DNA sequence may then be restricted to provide for a sequence encoding the entire gB precursor or fragments thereof. These sequences may then be inserted into an appropriate expression vector having appropriately positioned transcriptional and, as appropriate, translational signals. This can be achieved by filling in overhangs and providing for blunt-end ligation, by employing adapters, or the like.

It is of particular interest to introduce the gene in tandem with a gene capable of amplification. Convenient genes include the dihydrofolate reductase (dhfr) gene, which can be amplified by employing methotrexate, where the dhfr gene and flanking regions are reiterated; and metallothioneins which can be amplified with heavy metals, e.g., copper, or the like. The expression product construct can be introduced into an appropriate host by any convenient means, including transformation, transfection, calcium phosphate precipitation, etc. The host cells may then be stressed with the appropriate biocide at levels which select for amplification of the particular gene. The cells may then be cultured and grown to provide efficient production of the desired polypeptide.

Following the procedure described above, the polynucleotide sequence coding for gB2 from a HSV-2 strain 333, both precursor and mature, may also be isolated, cloned, and manipulated to provide a construct which may result in expression in one or more hosts. In view of the availability of fragments coding for gB1-Patton, these fragments may be used as probes for either localization of gB2 encoding DNA segments to specific HSV-2 restriction fragment clone(s) or isolation of gB2 mRNA from infected host cells. Conveniently, a plurality of probes may be employed coding for different regions of the gB1 gene. One selects for either positive DNA fragment(s) or abundant mRNA having approximately the right size which hybridizes to the probe(s). The mRNA may then be reverse transcribed to provide cDNA and/or may be used for hybridization to fragments of the HSV-2 genome to confirm their gB2 encoding function. Where necessary, more than one cloned fragment comprising portions of the gB2 structural gene may be manipulated and joined to provide the entire coding region and flanking region(s), as appropriate. The coding region may then be introduced into an expression vector.

### Recombinant Glycoprotein D

The preparation of recombinant gD1 is described in detail International Application No. PCT/US85/00587, which is International Publication No. WO 85/04587, published 24 October 1985. A brief description of the materials and methods used to make recombinant gD polypeptides follows. A detailed description of the preparation of recombinant gD2 is presented in the Experimental section below.

Polypeptides which are immunologically cross-reactive with naturally occurring glycoprotein D are produced in eukaryotic hosts, e.g., yeast and mammalian cells, such as CHO cells by recombinant DNA methodology. Production in eukaryotes provides the advantages associated with eukaryotic hosts, e.g., post-translational modification and/or secretion. The gD polypeptides may be produced from relatively short synthetic DNA fragments encoding for at least about 9 amino acids to provide haptens useful for eliciting an immune response specific for gD.

The gD DNA fragments may be of natural or synthetic origins. The natural gD gene of HSV-1 is located on the viral genome between the short internal repeat (IR_{S}) sequence and short terminal repeat (TR_{S}) sequence at the 3'-end thereof. Coding for the mature protein is found on an approximately 1.6kbp fragment located on a 2.9kbp SacI restriction fragment of the genome. The entire coding region for the mature protein is located within a HindIII-NruI fragment of the 2.9kbp SacI fragment. The naturally occurring gD gene may be employed with or without modification. Regions of the gene may be deleted and/or joined to other DNA fragments as desired. The gD DNA fragments may be inserted in expression vectors and expressed using similar materials and procedures as are described above for the expression of gB DNA. The preparation, cloning and expression of particular fragments of the naturally occurring gD gene are described in detail in the Experimental section hereinafter.

The following examples are offered by way of illustration and not by way of limitation. In the examples: Section 1 describes general procedures used to make the recombinant proteins; Section 2 describes the preparation of recombinant gB1; Section 3 describes the preparation of recombinant gB2; Section 4 describes the preparation of recombinant gD1; Section 5 describes the preparation of recombinant gD2; and Section 6 describes vaccine studies using mixtures of gB and gD polypeptides.

### EXAMPLES

### 1. Materials and Methods.

The HSV-1 strain Patton and HSV-2 strain 333 viable stocks are available from Dr. Richard Hyman, Hershey Medical Center, Hershey, Pennsylvania. These viruses can be propagated in Vero cells available from Dr. Evelyn Linnette, Viro Labs, Emeryville, California, or from the American Type Tissue Culture Laboratory, the propagation being performed in accordance with standard procedures. A library of HSV-1 Patton EcoRI DNA fragments (Kudler et al., Virology (1983) 124: 86-99) cloned in the EcoRI site of the plasmid pACYC184 (Chang and Cohen, J. Bacteriology (1978) 134:1141) can be obtained from Dr. Hyman or be independently prepared in accordance with conventional techniques. Two HSV-2 333 clones can also be obtained from Dr. Hyman, namely the HindIII fragments H and L inserted into the HindIII site of pBR322 (Sutcliffe, Nucleic Acids Research (1978) 5:2721).

The dhfr deficient CHO cell line was obtained from Dr. Y.W. Kan (University of California at San Francisco). This cell line was originally described by Urlaub and Chasin, Proc. Natl. Acad. Sci. USA (1980) 77:4216-4220. For nonselective conditions, these cells were grown in Ham's F-12 medium (available from Gibco, cat. no. 176) supplemented with 10% fetal calf serum, 100U/ml penicillin, 100µg/ml streptomycin and 150µg/ml L-proline. Selective media was DME supplemented with 10% dialyzed fetal calf serum plus penicillin, streptomycin and 150µg/ml L-proline. For methotrexate (MTX) selection, concentrated MTX stocks were prepared from MTX obtained from Lederle and added to the above DME selective media immediately before use.

### 1.1 Cloning.

All DNA manipulations were done according to standard procedures. See, Maniatis et al., Molecular Cloning, CSH (1982). Restriction enzymes, T4 DNA ligase, E. coli DNA polymerase I Klenow fragment, and other biological reagents were purchased from Bethesda Research Laboratories or other indicated commercial suppliers and used according to the manufacturer's directions. Double-strand DNA fragments were separated on 1% agarose gels and isolated by electroelution.

### 1.2 Isolation of RNA, Northern blot analysis and hybrid-selected translation.

Total RNA was prepared from HSV-1 or HSV-2 infected Vero cells at 6 hrs after infection with multiplicity of 10 virus per cell. Cell monolayers were washed, incubated with extraction buffer and processed as described (Pachl et al., Cell (1983) 33:335-344). Poly A⁺ RNA was prepared by passing 2mg total RNA over a 3ml column of oligo dT cellulose (obtained from Collaborative Research) in 500mM NaCl, 10mM Tris HCl pH 7.5, 1mM EDTA, 0.1% SDS, then washing the column in 100mM NaCl, 10mM Tris HCl pH 7.5, 1mM EDTA, 0.1% SDS and then eluting the poly A⁺ fraction with 10mM Tris HCl pH 7.5, 1mM EDTA, 0.1% SDS.

For Northern blot analysis, poly A⁺ RNA was denatured with glyoxal (McMaster et al., Proc. Natl. Acad. Sci. USA (1977) 74:4835-4838), fractionated by electrophoresis on 1% agarose gels, transferred to nitrocellulose paper (Thomas, ibid. (1980) 77:5201-5205) and hybridized with ³²P-labeled probes.

The details of the methods used for hybrid-selected translations have been described previously (Pachl et al., Cell (1983) 33:335-344). DNA filters were prepared using either 3µg of a 3.5kb Xho-Kpn fragment encoding gB or 2µg of a 3.0kb SstI-SstI fragment encoding HSV-1 gD. The filters were incubated with 40µg of poly A⁺ RNA from HSV-1 infected cells. Bound RNA was eluted and translated in a reticulocyte cell-free system (Pachl et al., J. Virol. (1983) 45:133-139). Translation products were analyzed on 12.5% SDS polyacrylamide gels (Laemmli, Nature (1970) 227:689).

### 1.3 DNA transfections.

Transformation of COS 7 cells (Gluzman, Cell (1981) 23:175-182) or dhfr deficient CHO cells (Urlaub and Chasin, (1980) supra) was carried out using the procedure of van der Eb and Graham (Methods in Enz. (1980) 65:826-839), as modified by Parker and Stark (J. of Virol. (1979) 31:360-369), except that carrier DNA was omitted. A calcium phosphate precipitate of plasmid DNA was prepared by mixing an equal volume of plasmid DNA, in 250mM CaCl₂, with an equal volume of 2x concentrated HEPES-buffered saline (2xHBS) added dropwise (1xHBS is 0.14M NaCl, 5mM KCl, 0.7mM Na₂HPO₄, 2.8mM glucose, 10mM HEPES pH 7.0). After about 20min incubation at room temperature, 1ml of the calcium phosphate-DNA suspension (containing 15µg DNA) was added to the media of cells, grown to 50% confluency on 10cm plates. After 6-8 hrs the DNA-containing media was removed and the cells were incubated with 15% glycerol-1xHBS for 4min. The cells were then grown in nonselective media (F12) for two days, after which the cells were split, i.e., subcultured, into selective media. Colonies of dhfr positive cells appeared after 10 days and were isolated after 14 days by removing the cells of a colony from a dish with a Pasteur pipette. The isolated cells were transferred to multiwell dishes for propagation.

### 1.4 In vivo labeling of cells and immunoprecipitation.

To label with ³⁵S-methionine, cells were grown to confluency in 3.5cm dishes, washed once with PBS (0.14M NaCl, 2.7mM KCl, 15.3mM Na₂HPO₄) and then 0.5ml of labeling media, DME (Dulbecco's Modified Eagle medium from Gibco, cat. no. 188G) without methionine plus 1% dialyzed fetal calf serum and 400µCi/ml ³⁵S-methionine (>1000Ci/mmole) was added per dish. The cells were incubated for appropriate times at 37°C. At the end of the labeling period, the media was removed and the monolayer washed once with PBS. For a "cold" methionine chase, the labeling media was replaced with DME containing 2.5mM methionine. For immune precipitation, cells were lysed in 0.1ml of lysis buffer: 20mM Tris-HCl pH 8, 100mM NaCl, 1mM EDTA, 0.5% Nonidet P40, 0.5% sodium deoxycholate, bovine serum albumin, 0.1% SDS, 1.0mM phenylmethylsulfonyl fluoride, 10mM benzamidine, 1% aprotenin obtained from Sigma Chemical Company. The cell lysate was scraped into tubes, briefly vortexed, and then held at 4°C for 5-10min. Cell debris was removed by centrifugation and the clarified lysate stored at -70°C.

For immunoprecipitations, cell lysates, 0.1ml, were precleared by incubation with normal serum for 30min at 4°C, then 50µl of a 20% solution of protein A Sepharose (PAS) (in lysis buffer) was added and incubation continued for 30min at 4°C with gentle rocking. The PAS was removed by centrifugation for 1min at 14,000xg and 5µl of HSV-1 polyclonal antibody (obtained from DAKO) or a gB-specific monoclonal antibody F3AB (obtained from Dr. John Oakes, University of South Alabama) was added. When the F3AB antibody was used, 0.1% SDS was omitted from the lysis buffer. After 30min at 4°C, 75µl of PAS was added and incubated as above. PAS-immune complexes were collected by centrifugation, washed 3x with lysis buffer lacking BSA and protease inhibitors and once with 0.12M Tris HCl pH 7.0. Immune precipitated proteins were released from PAS by boiling in SDS sample buffer, followed by analysis on 12% polyacrylamide gels. For immune precipitation of labeled proteins from cell media, the media was first clarified by centrifugation and then 1/10 volume of 10x lysis buffer was added and proteins were precipitated as described above.

### 1.5 Immunofluorescence.

To analyze expression of gB or gD in COS cells or CHO clones, cells, grown in slide wells, were washed 3x with PBS, fixed with 100% methanol at -20°C for 10min followed by 3 more PBS washes and one wash with PBS plus 5% goat serum (GS). The fixed cells were then incubated with the primary antibody (HSV-1 or HSV-2 polyclonal diluted 1/100 in PBS-5% GS) for 30min at 37°C. The cells were then washed 3x in PBS-5% GS and then incubated at 37°C for 30min with the second antibody, FITC-conjugated goat anti-rabbit IgG (Cappel), diluted 1/10 in PBS-5% GS. After 4 washes in PBS-5% GS, the slides were mounted with coverslips using 50% glycerol--100mM Tris HCl, pH 8 and observed in a Leitz microscope equipped with epifluorescent optics. Live cell immunofluorescence was carried out as described above except that the cells were initially washed once in PBS-5% GS directly followed by incubation with the first antibody. Before mounting with coverslips, the live cells were fixed with 5% formaldehyde in PBS. The fluorescein stained cells were photographed using a Kodak Ektachrome film (ASA 400).

### 1.6 ELISA Assay

The concentration of gB protein in CHO cell conditioned medium was measured by an indirect enzyme-linked immunosorbent assay (ELISA) using a preparation of purified recombinant gB as a standard. Aliquots of 50µl of F3AB antibody diluted 1:1000 in PBS were adsorbed to the wells of a 96-well polyvinyl chloride plate (Dynatech Laboratories, Inc.) by incubation for 1 hr at room temperature. Excess antibody was removed by 3 washes with PBS-5% GS, 50µl aliquots of media samples or the gB protein standard diluted in PBS + 1% GS were added to the wells and incubated for 1 hr at room temperature. The plates were then washed 3 times with PBS + 1% GS and followed by a third 1 hr incubation with 50µl of rabbit anti-HSV-1 polyclonal antibody (obtained from DAKO) diluted 1:100 in the same buffer. Excess secondary antibody was removed by 3 washes with PBS + 1% GS. Finally, 50µl of goat anti-rabbit horseradish peroxidase-conjugated antibody (Boehringer Mannheim) diluted 1:500 in PBS + 1% GS was added to each well for a 1 hr incubation. The wells were then washed once with PBS + 1% GS, followed by 8 washes with PBS and then developed with 50µl of 2,2'-azido-di[3-ethylbenz- thioazoline sulfonate] (Boehringer Mannheim) at a concentration of 1mg/ml in 0.1M citric acid, pH 4.0, 0.003% H₂O₂. The color reaction was stopped after 5 minutes by the addition of 50µl of 10% SDS and the absorbance was read at 414nm in a microtiter plate reader.

The concentration of gD protein was measured in similar fashion except that purified recombinant gD was used as a standard, and 8D2, a gD-specific monoclonal antibody (Rector et al., Infect. and Immun. (1982) 38:168-174) replaced F3AB.

### 2. Glycoprotein B1.

### 2.1 Isolation, cloning and characterization of the gB1 gene.

To isolate the gene for the glycoprotein gB1, DNA fragments spanning map coordinates 0.345 to 0.40 within the EcoRI F restriction fragment of the HSV-1 strain Patton (Skare and Summers, Virology (1977) 76:581-595) were subcloned in the plasmid pBR322. These fragments were prepared from the appropriate restriction digests of the EcoRI region in the plasmid pACYC184, separated by electrophoresis on a 1% agarose gel in TAE buffer (0.04M Tris-acetate, 0.002M EDTA) and electroeluted. The isolated fragments were ligated into pBR322 which had also been previously cut with the appropriate restriction enzyme and treated with alkaline phosphatase. A restriction map for the entire HSV-1 genome is shown in Fig. 1, and a more detailed map of the region which was subcloned is shown in Fig. 2. Referring to Fig. 1, the conventional map is shown in the first two lines (Roizman, 1979). The dotted line indicates the L-S junction. The restriction enzyme cleavage map for EcoRI for the prototype isomer arrangement is shown in the third line (Skare and Summers, 1977; Roizman, 1979) with the EcoRI fragment F denoted by the cross-hatched box. For HSV-2, the HindIII restriction map is shown in line 4 (Roizman, 1979) with the HindIII fragment H cross-hatched. One map unit corresponds to approximately 98.9 megadaltons or 148.9kbp of DNA for HSV-1 and 105.6 megadaltons or 160.5kbp of DNA for HSV-2.

Referring to Fig. 2, the restriction enzyme sites shown in the detailed map line (I) are E, EcoRI; B, BamHI; S, SalI; P, PstI, X, XhoI from DeLucca et al., 1983; N, NdeI; Xn, XmnI; V. EcoRV. The BstEII site mapped by DeLucca et al. at 0.355 is missing in this strain and there is a new PstI site at 0.357. Line II shows three plasmid subclones which encompass the gB1 coding region. They are pHS106, which extends from the BamHI site at 0.345 to the SalI site at 0.360; pHS107 which extends from the SalI site at 0.36 to the SalI site at 0.388; and pHS108 which is a BamHI fragment extending from 0.345 to 0.40 map units. Line III indicates three probes used for mRNA mapping of gB1; line IV indicates the fragment used for hybrid selection; and line V shows those probes used to locate the gB2 gene (see below). The additional restriction sites used to generate these fragments are Nc, NcoI; K, KpnI; and A, AluI.

To locate the gB1 coding region within the EcoRI F fragment, Northern blots of poly A⁺ mRNA isolated from HSV-1 infected Vero cells were probed with the DNA fragments indicated on the detailed map isolated from plasmids pHS106 and pHS107. When HSV-1 mRNA was probed with a 0.56kb PstI-SalI fragment isolated from pHS106, a 3kb mRNA was the major species detected. When the same blot was probed with a 0.49kb NcoI fragment, which maps about 1kb upstream from the PstI-SalI fragment, hybridization to a 3kb mRNA, the presumptive gB1 mRNA, was also detected. This suggests that the gB1 coding sequences extend at least 1kb to the left of the PstI-SalI fragment. The 3kb mRNA does not extend beyond the first XhoI site downstream from the PstI-SalI fragment, since the 0.5kb XhoI-XhoI fragment does not hybridize to this mRNA. The direction of transcription of the gB1 transcription unit is right to left (3' ← 5') as evidenced by hybridization of only the 5' → 3' oriented strands of the PstI-SalI and NcoI-NcoI fragments (cloned in M13) to the 3kb gB1 mRNA.

Hybrid selected translation was performed by hybridizing HSV-1 poly A⁺ mRNA with a 3.2kb KpnI-XhoI fragment, which encompasses the region indicated as encoding gB1. When the bound mRNA was eluted and translated in vitro, a 100kd protein, similar in size to gB1 from HSV-1 infected Vero cells, was detected. Confirmation of the identity of the 100kd protein was achieved by immunoprecipitation with a gB1-specific monoclonal antibody. Several other proteins were also detected by hybrid selection using the KpnI-XhoI fragment, probably the result of non-specific hybridization of mRNAs due to the high G+C content of the DNA. A similar pattern of proteins was seen when the same RNA was selected with a 3.0kb SstI-SstI DNA fragment encoding HSV-1 glycoprotein gD, except that the 100kd gB protein was not detected. This result indicates that gB is specific to the XhoI-KpnI fragment.

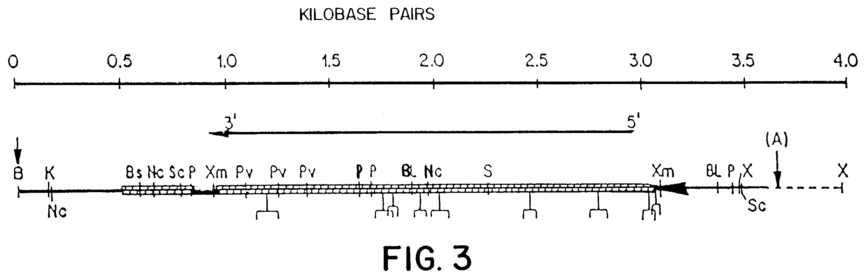

Fig. 3 is a restriction map of a 3.95kb DNA fragment, which extends from a BamHI restriction site at 0.345 to an XhoI site at 0.373 map units. The open reading frame for gB1 is indicated by the box and the direction of transcription is from right to left as shown. The actual coding region covers map units 0.348 to 0.367. The DNA sequence from the BamHI site to a non-unique AluI site at nucleotide number 3640 is shown with the AluI site indicated by the (A). The restriction sites shown include B, BamHI; Bl, BalI; Bs, BstEII; K, KpnI; Nc, NcoI; P, PstI; Pv, PvuII; S, SalI; Sc, SacI, X, XhoI; Xm, Xma3. Restriction sites are not shown for the right-hand end from the AluI site to the terminal XhoI site. Potential glycosylation sites and hydrophobic anchor and signal regions (solid box) in the product gB1 protein are noted.

The DNA sequence was determined from the BamHI site to a non-unique AluI site at nucleotide residue number 3640 using the M13 dideoxynucleotide synthesis method of Sanger. Both DNA strands across the coding region were sequenced. The entire DNA sequence was compiled from overlapping restriction fragments such that the sequence was read across all restriction fragment joints. Fig. 4 shows the DNA sequence for gB1 (line 3); the predicted amino acid sequence for gB1 is shown below the DNA sequence (line 4).

It should be noted that the amino acid sequence and DNA sequence for gB1 presented in Fig. 4 differs from that originally presented in Table 1 of International Publication No. WO 85/04587, published 24 October 1985. The DNA sequence in said Table 1 contains an error in that an additional nucleotide (G) is listed at position 607; this nucleotide has been deleted in Fig. 4, which presents the corrected DNA sequence. The amino acid sequence in said Table 1 was deduced from the incorrect DNA sequence presented therein; the sequence as presented in said Table 1 is incorrect because of the shift in reading frame due to the additional nucleotide. Fig. 4 presents the amino acid sequence based upon the corrected DNA sequence; the amino acid sequence in Fig. 4 has been confirmed by amino acid sequencing of the N-terminal region of gB1. This change in the deduced amino acid sequence also results in correction concerning the deduced position of the hydrophobic and hydrophilic regions, and the glycosylation sites in the gB1 molecule. The deductions based upon the corrected sequence are presented below.

Primer extension, using a 22bp oligonucleotide (residues 473-494) indicated that the 5'-end of gB1 mRNA was located at residue 188. the CAT and TATA transcriptional regulatory signals are presumptively at residues 55-62 and 125-131. Starting at the ATG at residues 438-440, there is an open reading frame of 2712 nucleotides which terminates at a TGA stop codon. Two presumptive polyadenylation signals are located in a 3'-non-coding region at residues 3166-3173 and 3409-3416.

The observed amino acid sequence is characteristic of a membrane protein. There is a very hydrophobic region near the carboxy terminus stretching from amino acid residue number 726 to 795, a 69-amino acid sequence which may span the membrane. At the N-terminus the first 30 amino acids are primarily hydrophobic. This hydrophobic amino acid domain precedes a region with a high concentration of charged or hydrophilic amino acids. The hydrophobic sequence at the N-terminus may serve as a secretory leader or signal sequence followed by processing signals for cleavage and removal of the secretory leader. The hydrophobic region near the C-terminus can serve as a transmembrane integration sequence for binding the protein to the cell membrane.

The sequence data is also suggestive that there are nine possible N-linked glycosylation sites as defined by the sequence asn-X-thr/ser (see also Fig. 3) within the hydrophilic, external domain. If the first 30 amino acids are removed by processing and each of the potential N-linked glycosylation sites are utilized with the addition of an average 2kd of carbohydrate per site, the molecular weight of the mature protein would be approximately 123Kd.

### 2.2 Expression of gB1 in mammalian cells.

To obtain expression of gB1 in mammalian cells, the plasmids pHS 112 and pHS 114 were transfected into CHO cells deficient in dhfr using the calcium phosphate precipitation method as described in Materials and Methods. E. coli HB101 strains transformed with plasmids pHS112 and pHS114 are on deposit at the ATCC, and have been assigned Accession Nos. 39650 and 39651, respectively. The construction of these strains is described in International Publication No. WO 85/04587, supra. Transfected cells are selected by employing a selective medium lacking thymidine, purines and glycine. Cells were isolated by removal with a Pasteur pipette and propagated in multiwell plates. A number of clones were isolated which were shown to produce gB by immunofluorescence and radioimmunoprecipitation employing an HSV-1 polyclonal antibody or a monoclonal antibody specific for gB. Three cell clones, pHS112-1, pHS112-9 and pHS112-23, were isolated which synthesize an intracellular form of the complete gB protein. The gB made in these cells appears to be glycosylated, since higher molecular weight forms can be detected after a one hour pulse, followed by a 5 hr chase, as compared to nonchased cells and about 10% of the gB is secreted into the media. Five cell clones (pHS114-5, pHS114-6, pHS114-7, pHS114-11 and pHS114-12) expressing the truncated gB were also analyzed and shown to also secrete some gB into the media. One of these cell lines, pHS114-7, was chosen for further amplification with MTX. Clones were initially selected at 0.01, 0.05, 0.10 and 0.3µM MTX. Three clones synthesizing high levels of gB, as detected by immunofluorescence, were isolated from the 0.3µM MTX selections. By radioimmune precipitation, these clones, pHS114-0.3µM-6, 23 and 25, synthesize 2-3 times more gB during a 1 hr labeling with ³⁵S-methionine than the unamplified clone, pHS114-7. Pulse chase experiments indicate that at least 8% of the gB synthesized in these clones during a 1 hr pulse is secreted extracellularly by 5 hr.

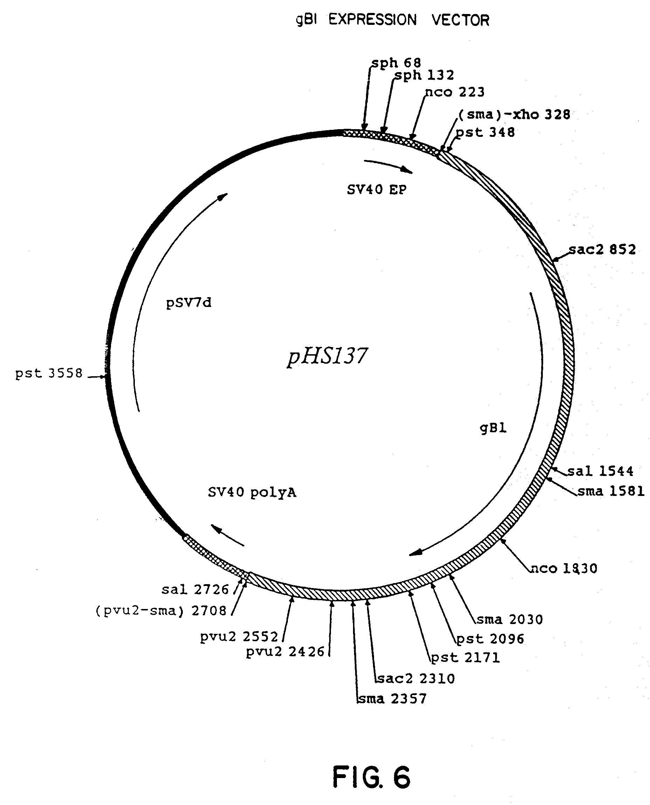

Expression was also achieved using the expression vector pHS137, a map of which is presented in Figure 6. Plasmid pHS137 encodes a truncated gB1 protein which is 690 amino acids in length after cleavage of the signal sequence.

pHS137 was constructed by digestion of pHS108 (described in Section 2.1) with XhoI and BamHI, followed by isolation of a resulting 3.5 kb fragment. The ends of this fragment were repaired to blunt with Klenow. The blunted XhoI-BamHI fragment was partially digested with PVUII, and DNA which migrated in gels as a 2098 bp band was iolated from the partial digest. The isolated XhoI-PVUII band was ligated into pSV7d which had been previously digested with SmaI, and the resulting DNA was used to transform E. coli. The resulting bacterial clones were screened for a plasmid with the proper orientation of the gB1 insert.

To obtain expression, pHS137 is cotransfected with the plasmid pADdhfr into dhfr deficient CHO cells. The resulting clones produce and secrete gB1. One such clone, pHS137-7-B-50 produces 6.91+/-1.53 µg/ml gB1 protein per 1-3 x 10⁷ cells in 24 hours in a T75 culture flask containing 10 ml of complete medium.

### 3. Glycoprotein B2.

The isolation, characterization, and cloning of the gB2 gene are described in International Publication No. WO 85/04857, supra.

### 3.1 Expression of gB2 in mammalian cells.

Expression of HSV-2 glycoprotein gB has been achieved in COS cells (transient expression) and in CHO cells (stable cell line secreting gB2) transformed with pHS210 alone or cotransformed with pHS210 and a second plasmid containing dhfr.

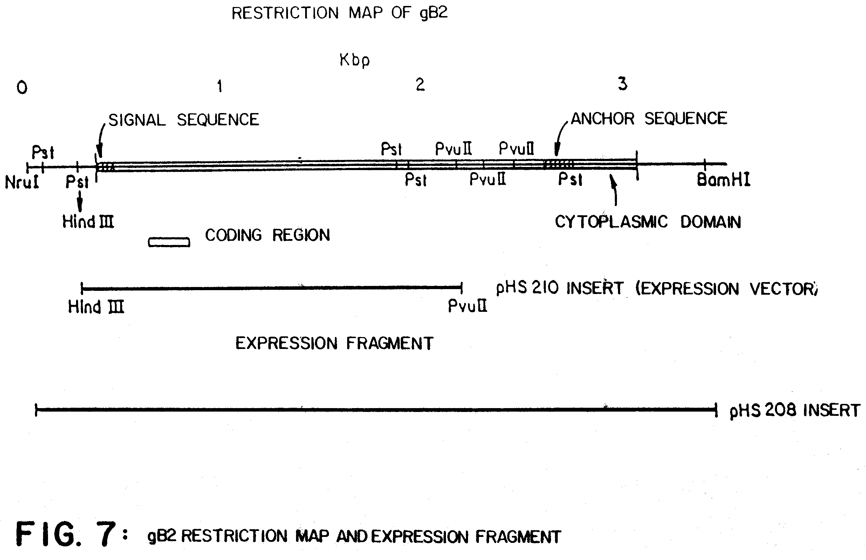

Plasmid pHS210 was constructed as follows: The entire gB2 gene was subcloned as a 3.8kb NruI-BamHI fragment in pBR322 to generate pHS208. See Fig. 7. The PstI site at the 5' end of the gene, 100bp to the right (downstream) of the NruI site, was changed to a HindIII site by in vitro mutagenesis in M13. A HindIII to PvuII fragment of 1.9kb was then inserted into pSV1, which was obtained by digestion of pSV1/dhfr with HindIII and BglII. See Fig 7; pSV1/dhfr has been described in PCT International Publication No. WO 85/04587. For this cloning step, pHS208 was cut with PvuII and the end repaired to blunt. The molecule was then cut with HindIII and the 1.9kb HindIII-(PvuII) fragment isolated by gel electrophoresis. Likewise pSV1/dhfr was cut with BglII, repaired to blunt, cut with HindIII and the 4.85kb HindIII-(BglII) vector fragment isolated by gel electrophoresis. These two fragments (1.9kb and 4.85kb) were ligated together to generate pHS210 - the expression plasmid (Fig. 7).

Plasmid pHS210 was used directly to transform COS cells. Expression was detected by immunofluorescence using a gB specific monoclonal antibody, F3AB, and also using a commercially available polyclonal anti HSV-2 antibody (DAKO) as the primary antibody screen. Secretion of gB2 into the medium was detected by a gB2-specific ELISA. For this purpose, plates were coated with the monoclonal antibody. Samples of cell culture medium were added to coated plates, then bound gB2 was detected with the rabbit anti HSV-2 polyclonal antibody (DAKO) followed by horseradish conjugated goat antirabbit IgG.

For CHO cell transformation plasmid pHS210 was used along with a second plasmid containing dhfr as a selective marker (Fig. 7) in a cotransfection protocol. Following transfection and propagation in selective media, approximately 100 dhfr⁺ clones were isolated and screened for synthesis and secretion of gB2 using an ELISA assay in which ELISA plates were coated with F3AB specific monoclonal antibody. Clone pHS210 #3-1, which had the highest levels of gB secretion, was chosen for further characterization of the gB2 polypeptide. The gB2 protein was detected by labeling with [³⁵S]-methionine followed by radio immunoprecipitation. After a 1 hr pulse, diffuse doublet bands corresponding to polypeptides of 79kd and 84kd were detected intracellularly. These proteins are larger than the 68,991 dalton size predicted for the 637 residue truncated gene product, and they presumably correspond to partially glycosylated precursors. After a 5 hr chase, no gB2 was detected intracellularly, and an 89kd polypeptide was detected in the medium. The size of the mature, fully glycosylated gB2 secreted into the media of clone pHS210 #3-1 is somewhat smaller than the 100kd gB1 secreted by pHS114-6 due to the removal from pHS210 of the coding sequence for 94 amino acids included in the gB1 plasmid.

### 4. Glycoprotein D1.

### 4.1 Construction of mammalian expression vectors for gD1

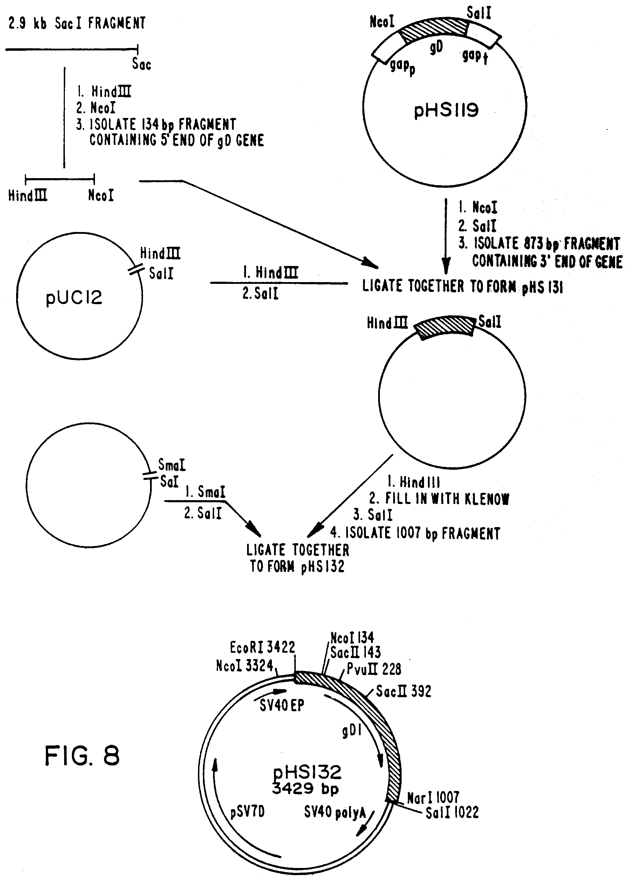

A library of EcoRI fragments of HSV-1, strain Patton, cloned into the EcoRI site of pBR322 was made by Dr. Richard Hyman, Hershey Medical Center, Hershey, PA. The gD1 gene is entirely contained within a 2.9kb SacI fragment within the EcoRI fragment of clone H from this library. Clone H, containing a 15kb EcoRI insert, was obtained from Dr. Hyman. The 2.9kb fragment was purified by gel electrophoresis and then digested to completion with HindIII and NcoI. The 5' end of the gD gene, consisting of 74bp of 5' untranslated sequences plus 60bp coding for the amino terminal 20 amino acids, was gel isolated as a 134bp fragment. The 3' end of the gD gene was obtained by digestion of pHYS119 (see International Publication No. WO85/04587, supra) with NcoI and SalI and isolation of the 873bp fragment. These two fragments (5' and 3' ends) were ligated together with the plasmid pUC12 which had previously been digested with HindIII and SalI. The pUC12 vector is commercially available from Pharmacia and P-L Biochemicals, the resulting plasmid was designated pHS131. The plasmid pHS131 was digested with HindIII, the 5'-4 base pair overhang was filled in with Klenow polymerase and then digested with SalI. The 1007bp fragment containing the gD gene was gel isolated and ligated into the plasmid pSV7d which had previously been cut with SmaI plus SalI. The plasmid pSV7d is described below. The resulting expression vector is designated pHS132. Its derivation is outlined in Fig. 8.

The plasmid encodes 315 amino acids of gD1 protein including a 25 amino acid signal sequence out of a total of 399 amino acids for the complete protein. The protein has been truncated at the carboxyl terminus and lacks 84 amino acids including the hydrophobic membrane anchor domain and the cytoplasmic domain such that the resulting protein is secreted into the medium.

The plasmid pSV7d was constructed as follows: the 400bp BamHI/HindIII fragment containing the SV40 origin of replication and early promoter was excised from SVgtI (Mulligan, R., et al., J. Mol. Cell Biol. (1981) 1:854-864) and purified. The 240bp SV40 BclI/BamHI fragment containing the SV40 poly A addition site was excised from pSV2/dhfr (Subramani et al., J. Mol. Cell Biol. (1981) 1:854-864) and purified. The fragments were fused through the following linker: 
Stop Codons
1 2 3
5'-AGCTAGATCTCCCGGGTCTAGATAAGTAAT-3'
TCTAGAGGGCCCAGATCTATTCATTACTAG
HindIII BglII SmaI Xba BclI overhang.
This linker contains five restriction sites, as well as stop codons in all three reading frames. The resulting 670bp fragment (containing the SV40 origin of replication, the SV40 early promoter, the polylinker with stop codons and the SV40 polyadenylation site) was cloned into the BamHI site of pML, a pBR322 derivative with about a 1.5kb deletion (Lusky and Botchan, Cell (1984) 36:391), to yield pSV6. The EcoRI and EcoRV sites in the pML sequences of pSV6 were eliminated by digestion with EcoRI and EcoRV, treated with Bal31 nuclease to remove about 200 bp on each end, and finally religated to yield pSV7a. The Bal31 resection also eliminated one BamHI restriction site flanking the SV40 region, approximately 200 bp away from the EcoRV site. To eliminate the second BamHI site flanking the SV40 region, pSV7a was digested with NruI, which cuts in the pML sequence upstream from the origin of replication. This was recircularized by blunt end ligation to yield pSV7b.

pSV7c and pSV7d represent successive polylinker replacements. Firstly, pSV7b was digested with StuI and XbaI. Then, the following linker was ligated into the vector to yield pSV7c: 
BglII EcoRI SmaI KpnI XbaI
5'-AGATCTCGAATTCCCCGGGGTACCT
TCTAGAGCTTAAGGGGCCCCATGGAGATC
Thereafter, pSV7c was digested with BglII and XbaI, and then ligated with the following linker to yield pSV7d:

BglII EcoRI   SmaI XbaI  BamHI SalI
5'-GATCTCGAATTCCCCGGGTCTAGAGGATCCGTCGAC
      AGCTTAAGGGGCCCAGATCTCCTAGGCACGTGATC

### 4.2 Expression of gD1 in mammalian cells.

Expression of gD1 from plasmid pHS132 has been demonstrated in many experiments. First, specific immunofluorescence was observed in COS 7 cells following transfection using the methods described previously and using a commercially available rabbit sera against HSV-1 (DAKO) for detection. Second, stable CHO cell lines secreting gD1 were established. The expression levels were analyzed by ELISA and verified by radioimmunoprecipitation of pulse labeled and chased cell lysates and media. Third, gD1 was purified from the media of roller bottle cultures of the CHO cell line D64 by sequential steps of ammonium sulfate precipitation, immunoaffinity chromatography and ultrafiltration. For the affinity chromatography the gD monoclonal antibody 8D2 described in Rector et al. (1982) supra. linked to cyanogen bromide activated Sepharose 4B was employed.

### 5. Glycoprotein D2.

### 5.1 Construction of mammalian expression vectors for gD2.

The HindIII L fragment of HSV-2 strain 333 was cloned in pBR322 by Dr. Richard Hyman as noted in the reference Kudler et al., Virology (1983) 124:86-99. The gene for the glycoprotein gD2 had been mapped to the short unique region of the virus between 0.90-0.945 map units by Ruyechan et al., J. Virol. (1970) 29:677-697, a region covered by the HindIII L fragment as shown in the genomic map of Roizman, B., Ann. Rev. Genet (1979) 13:25-57. The DNA sequence of the gD2 gene has been published by Watson, Gene (1983) 26:307-312.

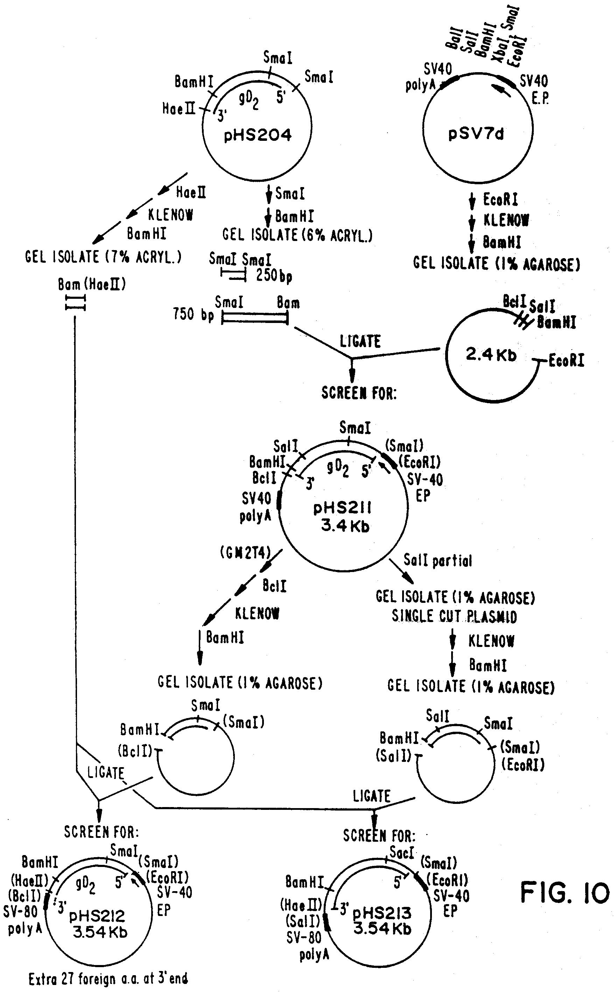

The HindIII L fragment cloned in pBR322 was obtained from Dr. Richard Hyman and the restriction map shown in Fig. 9A determined. The gene for gD2 was found to lie on a 2.4kb XhoI fragment by probing Southern blots of restriction digests of the HindIII L fragment with the 2.9kb SacI fragment encoding gD1. A map of the XhoI fragment and the position of the gD2 gene is shown in Fig. 9B. The 2.4kb XhoI fragment was cloned in a pBR322 derivative vector containing an XhoI site to generate plasmid pHS204. Three different gD2 expression vectors, plasmids pHS211, pHS212, and pHS213 were constructed as follows and as diagrammed in Fig. 10. The plasmid pHS211 encodes the first 305 amino acids of gD2 including the signal sequence. For its construction pHS204 was cut with SmaI and BamHI and two restriction fragments were gel isolated: a 250bp SmaI fragment containing the 5' end of the gene including 82bp of 5' untranslated sequence and the 3' adjacent 746bp SmaI-BamHI fragment containing an interior portion of the gene. The mammalian cell expression vector pSV7d (described in Section 4.2) was cut with EcoRI, the 5' 4bp overhang repaired to blunt with Klenow polymerase and then cut with BamHI. The two fragments from pHS204 were ligated into the digested pSV7d and bacterial transformants were screened for the appropriate orientation of the SmaI fragment to generate the vector pHS211.

The plasmid pHS212 which encodes 352 amino acids of gD2 or 47 additional residues beyond those present in pHS211 was constructed by the digestion of pHS204 with HaeII and repairing the ends to blunt with Klenow polymerase followed by digesting with BamHI. A 141bp (HaeII) (the parenthesis intends the terminus has been filled in) to BamHI fragment was gel isolated. The plasmid pHS211 was transferred into the E. coli strain GM272 (dam⁻) and plasmid DNA prepared, which was then restricted with BclI followed by blunt end repair with Klenow polymerase then digestion with BamHI. The large vector fragment (about 3.4Kb) was gel isolated and ligated together with the 141bp (HaeII)-BamHI fragment to generate the plasmid pHS212. The fusion of gD2 sequences to plasmid vector sequences at the 3' end of the gene results in the addition of 27 codons of nonsense DNA to the 3' end of the gD2 gene. To eliminate these nonsense sequences the plasmid pHS213 was constructed by partial digestion of pHS211 with SalI and gel isolation of the single cut plasmid which was then repaired to blunt with Klenow polymerase and digested with BamHI. The (HaeII) to BamHI fragment of 141bp from pHS204 was ligated into the linearized, pHS211 to generate the plasmid pHS213.

### 5.2 Expression of gD2 in mammalian cells.

The expression of gD2 in mammalian cells was first assayed by transfection of COS 7 cells with pHS211, pHS212 and pHS213 for transient expression. Expression of gD2 was detected both by immunofluorescence and by capture ELISA analysis of COS 7 conditioned media using a rabbit anti-HSV-2 antibody for the immunofluorescence and a gD type common antibody, 8D2 (Rector et al., (1982) supra.), for the capture antibody in the ELISA.

Permanent CHO cell lines were then established by transfection with the plasmids pHS211 or pHS213 with Ad dhfr and selection for dhfr acquisition and screening by ELISA for gD2 expression.

### Description of Ad-dhfr:

The plasmid bearing the dhfr gene was constructed by fusing the major late promoter from adenovirus-2 (Ad-MLP, map units 16-17.3) to the mouse dhfr cDNA at the 5' end. DNA coding for the intron for SV40 small t Antigen and the SV40 early region polyadenylation site was obtained from pSV2-neo, described in Southern and Berg, J. Mol. Appl. Genet. (1982) 1:327-341, and fused to the 3' end of the dhfr cDNA. These three segments were subcloned into pBR322 to obtain the plasmid Ad-dhfr. This plasmid is functionally similar to the dhfr plasmid described in Kaufman and Sharp, Molec. and Cell Biol., (1982) 2:1304-1319.

### 6. Therapeutic Treatment with gB-gD Vaccine.

### 6.1. Effect of Recombinant HSV glycoprotein vaccines administered after primary infection on subsequent recurrent herpetic diseases in guinea pigs.

Female Hartley guinea pigs were intravaginally inoculated with 5 x 10⁵ pfu HSV-2 MS strain on day 1. Animals were treated with acyclovir (5mg/ml) from days 1-10 by addition to the drinking water. Acyclovir reduces the severity of primary infection and thus the mortality, incidence of secondary bacterial infection and incidence of genital scarring. The use of acyclovir during primary infection has been shown to have no impact on the course of the disease after the cessation of treatment for the guinea pig (Bernstein et al., Virology, (1986) 67:1601). After recovery from primary infection, the animals were immunized with HSV-2 total glycoprotein preparation (gP2), with a mixture of recombinant gB1 and gD1 (HSV-1 gB + gD) or received no treatment. Treatment groups are shown below:

| Group | Treatment | Dose | Adjuvant | Route | N |
|---|---|---|---|---|---|
| I | None | None | None | None | 11 |
| II | HSV-1 gB+gD | 25µg +25µg | Freund's | Footpad | 11 |
| III | gP2 | 50µg | Freund's | Footpad | 11 |
| IV | Control, Adjuvant only | None | Freund's | Footpad | 9 |

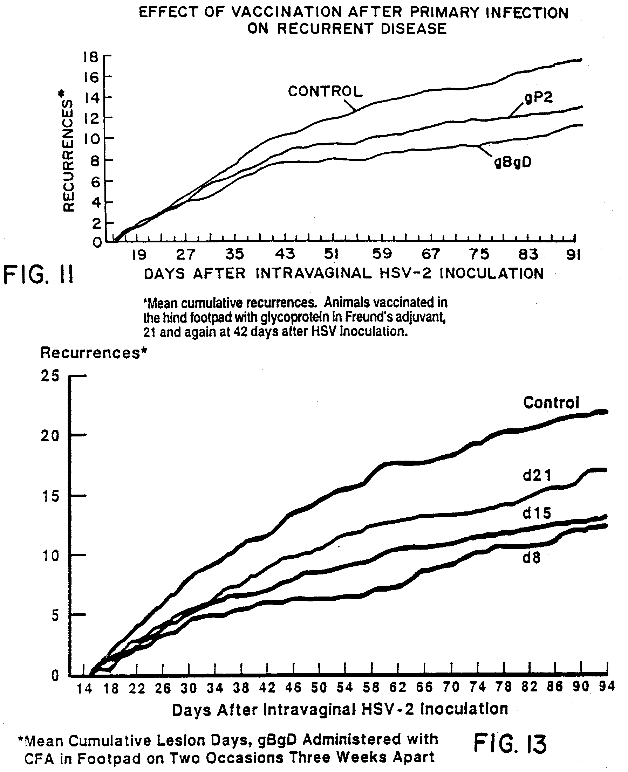

Animals were immunized on day 21 and again on day 42 by injection of the vaccines into the hind footpads. Both recombinant proteins gB1 and gD1 were produced in mammalian cells as previously described. Results are reported in Table 1 and Figure 11.

The results show that the pattern of recurrent herpetic disease was the same for Groups I and IV, hence these groups were pooled for analysis (control, n=20).

**TABLE 1**

| Group | N | Days Lesions Observed (Mean ± SE) | Recurrent Episodes (Mean ± SE) | Percent Severe Recurrences^{c} | Days/Episodes |
|---|---|---|---|---|---|
| Control | 20 | 15.9 ± 1.5 | 9.0 ± 0.7 | 19.4 ±2.9 | 1.77 |
| gB + gD | 11 | 9.0 ± 1.6^{b} | 6.6 ± 1.0^{a} | 7.1 ± 2.2^{b} | 1.36 |
| gP2 | 11 | 11.0 ± k0.9^{b} | 7.3 ± 0.7^{a} | 12.1 ± 3.1^{b} | 1.51 |

| | | | | | |
|---|---|---|---|---|---|
| ^{a} Glycoproteins (50µg) administered with complete Freund's adjuvant in the hind footpad 21 and again 42 days after intravaginal HSV-2 challenge; recurrences scored day 21 through 92. | | | | | |
| ^{b} Significantly different from control (p <0.05). | | | | | |
| ^{c} Percent recurrences with two or more herpetic lesions. | | | | | |

Results shown in Table 1 and Fig. 11 indicate that vaccination with the recombinant glycoproteins has a significant impact on the frequency of recurrent disease. In addition, the gB + gD combination is better than the mixture of natural glycoproteins.

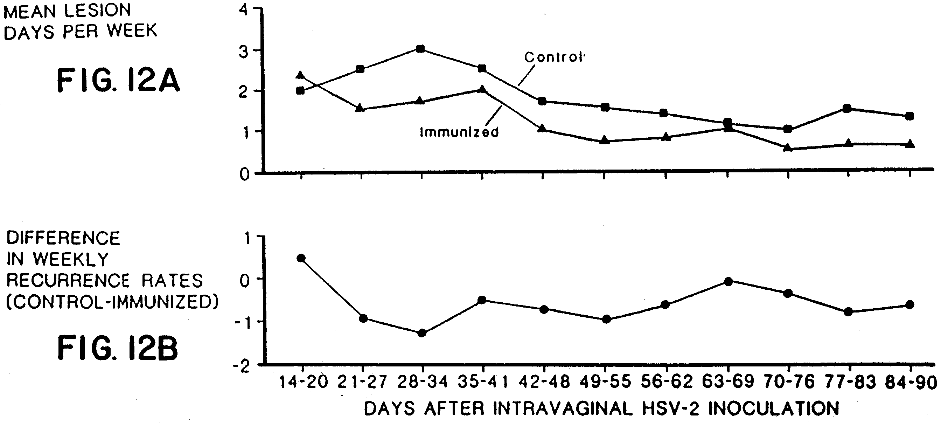

The rate of recurrent disease as measured by the number of lesion days occurring within a specified time is an assessment that considers both the frequency and the duration of recurrent episodes. Figure 12A shows the rate of recurrent herpetic infections, expressed as the mean number of days per week that herpetic lesions were noted. The immunized group includes both gBgD and gP-2 vaccinated animals. As shown in Figure 12A, the rate of recurrent disease (lesion days per week) declined in all groups as the period of evaluation became more remote to the initial infection, but the rate of decline was greater in the vaccinated animals. The difference in the rates of recurrent herpetic infections between control animals and immunized animals is shown in Figure 12B. As seen in Figure 12B, the effect of glycoprotein immunization on the rate of recurrent disease appeared to have been established following the first immunization dose rather than after the second dose, as might have been deduced from Figure 11.

### 6.2. Effect of recombinant HSV glycoprotein vaccines administered after primary infection on the host immune response.

The effect of post-infection glycoprotein administration on the host immune response was determined by measuring anti-HSV antibodies produced by the infected animals prior to infection, and after immunization with HSV glycoprotein vaccines.

The animals were inoculated with HSV-2 ms strain, treated with acyclovir, and treated with HSV glycoprotein vaccines as described in Section 6.1. Sera from the animals was collected on days 41 and 95. Anti-HSV antibodies in the sera was measured by ELISA, essentially as described in Pachl, C., et al, J of Virology (1987) 61:315-325, which is the procedure described in Section 1.6. The capture antigens included HSV-1 glycoprotein mixture (gP-1), HSV-1 glycoprotein D (gD-1) or HSV-2 glycoprotein D (gD-2).

The effects of HSV glycoprotein vaccine administration on anti-HSV antibody titers is shown in Table 2, where the data is expressed as the geometric mean. Antibody was not detected in sera collected prior to HSV inoculation. As seen in Table 2, in the untreated control animals anti-HSV antibody titers were greater on day 41 than on day 95. In contrast, glycoprotein treated animals generally exhibited rising titers through day 95, and vaccination with HSV glycopoteins resulted in significant increases in anti-HSV antibody titers (p < .05) compared to the untreated controls. Moreover, whereas treatment with the gP-2 mixture produced a 1.4 to 7 fold increase in antibody titers, treatment with recombinant HSV-1 gBgD vaccine resulted in a 9 to 31 fold elevation in titers compared to control values. Thus, the administration of HSV glycoproteins to animals, and particularly recombinant HSV glycoproteins gBgD, augments the host immune response and, as shown above in Section 6.1, reduces the frequency and severity of recurrent HSV disease.

**Table 2**

| Effects of HSV Glycoprotein Vaccine Administration after Recovery from Initial Genital Herpes on Anti-HSV Antibody Titers in Guinea Pigs | | | | | | |
|---|---|---|---|---|---|---|
| Treatment | Anti-HSV Antibody | | | | | |
| | gP-1 Antibody | | gD-1 Antibody | | gD-2 Antibody | |
| | Day 41 | Day 95 | Day 41 | Day 95 | Day 41 | Day 95 |
| Untreated | 548 | 474 | 32.5 | 14 | 65 | 48 |
| Adjuvant only | 818 | 754 | 22 | 34 | 25 | 68 |
| gP-2 | 2796 | 3343 | 177 | 152 | 91 | 297 |
| gBgD | 9891 | 14881 | 2444 | 4864 | 606 | 2391 |

### 6.3. Effect of adjuvants on the immune response induced by HSV glycoprotein vaccines containing gD1.

Several adjuvants were examined to determine their effect on the efficacy of immunotherapeutic treatment with HSV glycoprotein vaccines. The adjuvants tested were complete Freund's adjuvant (CFA), alum, and N-acetylmuramyl-L-alanyl-D- isoglutaminyl-L-alanine-2-(1'-2'-dipalmitoyl-sn- glycero-3-hydroxyphosphoryloxy)-ethylamine referred to as MTP-PE).

The adjuvant effects were determined by measuring the amount of anti-gD1 antibodies resulting from administration of gD1 containing vaccines which were also comprised of the various adjuvants. The vaccines containing gD1 alone serve as a model for gBgD containing vaccines, since the adjuvant effect is not expected to be specific to the type of HSV glycoprotein in the vaccines.

In the following studies gD1 was synthesized from pHS132 and isolated as described in Section 4.2. Female guinea pigs were given three footpad immunizations consisting of 35 µg gD1 and various adjuvant formulations at three week intervals. One week after the second immunization, and one, five, nine and thirteen weeks after the third immunization the animals were bled and anti-gD titers were determined by ELISA, as described in Section 1.6.

The results presented below are indicative that the most promising of the adjuvants tested is MTP-PE, since it consistently produce high anti-gD1 titers in experimental animals. These levels were equivalent to those seen with CFA, although the titers were not maintained for as long a period of time as with CFA.

### 6.3.1. Comparison between CFA, and alum.

The animals were immunized with vaccines containing gD1 and either CFA or alum. The effect of the adjuvants on anti-gD titers is shown in Table 3, where the data is expressed as the geometric mean. As seen in Table 5, the most effective adjuvant was CFA. The highest antibody titers of the longest duration were seen in the group immunized with the CFA vaccine. The effect of the other adjuvant is expressed as the percent of the titer obtained with CFA. The lowest anti-gD1 titers were obtained using as adjuvant a 10% alum suspension.

**Table 3**

| HSV Adjuvant Study Mean ELISA Titers | | | | | | | |
|---|---|---|---|---|---|---|---|
| Experiment A Adjuvant | Number | Bleed 1 | % of CFA | Bleed 2 | % of CFA | Bleed 3 | % of CFA |
| CFA/IFA | 6(5) | 13709 ± 2161 | 100 | 15066 ± 2878 | 100 | 16834 ± 5527 | 100 |
| Alum | 4 | 1199 ± 467 | 9 | 1353 ± 318 | 9 | 1118 ± 211 | 7 |

### 6.3.2. Comparison of CFA, nor-MDP, and MTP-PE.

The animals were immunized with vaccines containing gD1 and either CFA, nor-MDP, and MTP-PE. The MTP-PE was encapsulated in liposomes, and this latter adjuvant was administered both with exogenous gD1, and with gD1 incorporated into the liposomes. Liposomes were prepared by vortexing synthetic phosphatidylcholine, phosphatidylserine and MTP-PE (or MTP-PE & gD1) at a ratio of 175:75:1 in suspension medium (sterile, isotonic Dulbecco buffer pH 7.2, without Ca⁺⁺ and Mg⁺⁺ salts). As seen in Table 4, immunization with vaccine containing CFA still yielded the highest anti-gD1 mean titer. The titers obtained with nor-MDP ranged from 44 to 74% of the mean titers obtained with the group immunized with CFA. The mean titers obtained with MTP-PE and exogenous gD1 were somewhat lower than that obtained with nor-MDP, with a range from 32 to 72% of those obtained with CFA. The very low titers obtained with MTP-PE and liposome encapsulated gD1 may be due to the very low levels of gD1 in the encapsulated form. The dose of encapsulated gD1 was only about 7% of the exogenous dose. This low dosage was due to the very low efficiency of incorporation of gD1 into liposomes, which may have been caused by the size of the antigen. Alternative formulations of liposomes could lead to more efficient incorporation of the antigen.

**Table 4**

| HSV Adjuvant Study Mean ELISA Titers | | | | | | | |
|---|---|---|---|---|---|---|---|
| Experiment B Adjuvant | Number | Bleed 1 | % of CFA | Bleed 2 | % of CFA | Bleed 3 | % of CFA |
| CFA/IFA | 7(6) | 8009 ± 1130 | 100 | 13962 ± 2304 | 100 | 8298 ± 896 | 100 |
| nor-MDP squalene/arlacel | 7(6) | 3519 ± 905 | 44 | 10387 ± 2946 | 74 | 5759 ± 920 | 69 |
| MTP-PE-Liposome | 7(6) | 4051 ± 891 | 51 | 9989 ± 1161 | 72 | 2653 ± 457 | 43 |
| MTP-PE-gD-Liposome | 7(6) | 415 ± 218 | 5 | 1656 ± 175 | 12 | n.d. | n.d. |

### 6.3.5. Comparison of different formulations containing MTP-PE.

The animals were immunized with gD1 containing vaccines formulated with MTP-PE in a high oil delivery system (Squalene/Arlacel), and MTP-PE in a low oil delivery system. The low oil formulation of MTP-PE contained 4% Squalene and 0.008% Tween 80. The gD1 antibody titers obtained with these adjuvant formulations are shown in Table 5.

As seen in Table 5, the MTP-PE formulation was effective as an adjuvant, even when used as the only constituent in the low oil-detergent formulation. [It was also an effective substitute for the CWS component in RIBI, moreover, compared to RIBI its effectiveness increased with time. At the third bleed, the titers obtained with MTP-RIBI were twice that obtained with RIBI.] After the first bleed, the MTP-PE low oil formulation showed a higher titer than the high oil delivery system (Squalene/Arlacel).

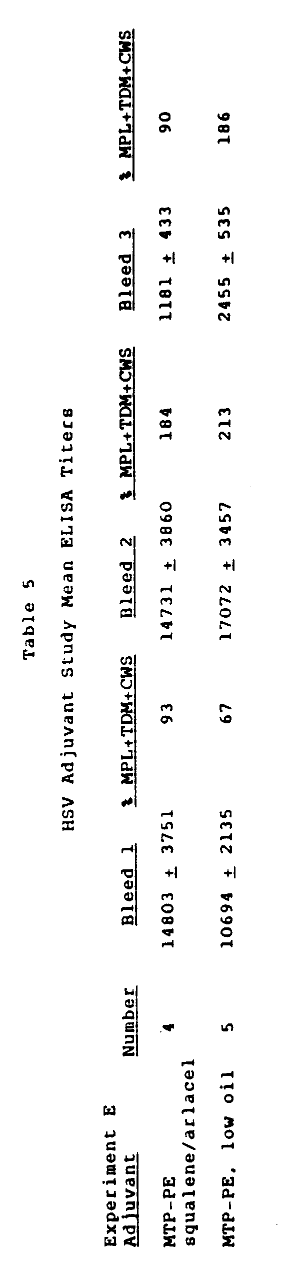

### 6.4. Therapeutic Studies: The effect of adjuvant, site of administration, and timing of administration on vaccine efficacy in preventing subsequent recurrent herpetic disease in guinea pigs.

The gBgD vaccine consisted of 25 µg each of recombinant gB1 and gD1 purified to approximately 70-80% homogeneity as judged by SDS-PAGE. The recombinant gB1 protein was a 50:50 mixture of gB1 prepared from cell line pHS113-9-10-21 and pHS137-7-B-50; these cell lines are CHO cell lines which harbor the vectors pHS113 and pHS137, respectively. The descriptions for the preparation of pHS113 and pHS137 are in Section 2.2. The gB protein was purified as described in Pachl et al, J of Virology, 61:315-325 (1987), which is essentially as described in Section 2.2. The gD1 was prepared as described in Section 4.2, except that during purification by affinity chromatography the anti-gD1 monoclonal antibody C4D2 replaced 8D2.

The present example compares the efficacy of two adjuvants, nor-MDP and CFA. The adjuvant nor-MDP was used at 50 µg/dose emulsified with 50% squalene/arlacel and the antigen.

The present study also compares two routes of administration, i.e., administration into footpads with administration which is intramuscular or subcutaneous. Finally, it compares various times of administration on the prevention of subsequent recurrent herpetic disease in guinea pigs. The experimental design is shown in Table 6.

**Table 6**

| Group | N | Treatment | | | |
|---|---|---|---|---|---|
| | | Immunogen | Adjuvant | Route | Day^{c} |
| 1 | 11 | none | | | |
| 2 | 13 | none^{a} | | | |
| 3 | 10 | gBgD | CFA | FP | 15,35 |
| 4 | 12 | gBgD | CFA | FP | 21,42 |
| 5 | 10 | gBgD^{a} | CFA | FP | 21,42 |
| 6 | 11 | gBgD | none | IM/SC | 21,42 |
| 8 | 10 | gBgD | nor-MDP | IM/SC | 21,42 |
| 9^{b} | -- | -- | -- | -- | -- |
| 10 | 10 | gBgD | none | FP | 21,42 |
| 12 | 11 | gBgD | nor-MDP | FP | 21,42 |
| 13 | 6 | gBgD | CFA | FP | 8,28 |

| | | | | | |
|---|---|---|---|---|---|
| ^{a} Daily vaginal swabs done d22-d100 to titer virus and assess asymptomatic shedding. | | | | | |
| ^{b} Group 9 was eliminated. | | | | | |
| ^{c} Day of administration of vaccine post-infection with initial virus exposure on day 1. | | | | | |

Female Hartley guinea pigs weighing 350-400 g were intravaginally inoculated with 5.7/log₁₀ pfu of HSV-2 strain MS on day 1. Animals were confirmed to be infected by recovery of HSV from vaginal swab samples collected 24 hr after intravaginal inoculation. The clinical course of initial infection was monitored and quantitated by a gential skin lesion score as described in Stanberry et al, J Infec Dis (1987) 155:914. After recovery from initial infection animals were randomized for the treatment groups shown in Table 10. Animals were examined daily for evidence of recurrent disease from days 11 to 100 after the resolution of the acute disease. Lesion days are defined as days on which recurrent lesions are observed, severe recurrences are days when more than one vesicle is noted, and episodes are the occurrence of a new lesion following a lesion free day.

The results obtained from analyses of the animals on days 22-76 are presented in Table 7. The data in Table 9 suggests that for IM injection, nor-MDP is an effective adjuvant; this is reflected in a lower total number of lesion days, a smaller percent of severe recurrences, and a diminishment in the total number of herpetic episodes. Moreover, the vaccine containing nor-MDP and administered IM appeared to be as effective as the vaccine containing CFA and administered in the footpads.

**Table 7**

| Effect of HSV-1 gBgD Vaccine Administered After Intravaginal HSV-2 Inoculation on Pattern of Recurrent Genital Herpes Preliminary Analysis - Days 22-76 | | | | | |
|---|---|---|---|---|---|
| Group # | Treatment | N | Total Lesion Days* | Percent Severe Recurrences* | Total Episodes* |
| 1 | Untreated | 11 | 16.7 ± 2.1 | 29.0 ± 5.7 | 8.9 ± 0.8 |
| 13 | gBgD-Day 8-CFA-FP | 5 | 9.0 ± 2.2 | 14.2 ± 3.9 | 5.8 ± 0.9 |
| 3 | gBgD-Day 15-CFA-FP | 9 | 10.0 ± 3.0 | 19.5 ± 7.4 | 6.2 ± 1.4 |
| 4 | gBgD-Day 21-CFA-FP | 11 | 11.6 ± 2.0 | 24.2 ± 3.2 | 7.3 ± 1.1 |
| 6 | gBgD-Day 21-No-Adj-IM | 10 | 14.2 ± 2.0 | 25.1 ± 5.3 | 8.0 ± 0.9 |
| 8 | gBgD-Day 21-nor-MDP-IM | 8 | 10.0 ± 1.8 | 20.5 ± 4.3 | 6.4 ± 1.1 |
| 10 | gBgD-Day 21-No Adj-FP | 10 | 14.5 ± 2.1 | 20.3 ± 3.1 | 8.9 ± 1.2 |
| 12 | gBgD-Day 21-nor-MDP | 9 | 14.8 ± 1.9 | 25.4 ± 5.0 | 8.2 ± 0.7 |

| | | | | | |
|---|---|---|---|---|---|
| *Mean ± SE | | | | | |

The local reactions resulting from injection of the vaccines containing the various adjuvants was also monitored, and the results are shown in Table 8. The incidence of local erythema and induration at the site of injection was the same for vaccines containing nor-MDP. Moreover, based upon the local reactogenicity, nor-MDP appears to be acceptable for use in vaccines.

**Table 8**

| Reactions Summary | | | | | | | | |
|---|---|---|---|---|---|---|---|---|
| Vaccination #1 | Group | N | ΔT°a | Erythema | Induration | Vaccine | Adjuvant | Route |
| | 1^{d} | 11 | ND^{b} | -^{c} | - | O | O | O |
| | 4^{d} | 12 | .08±.40 | - | - | gBgD | CFA | FP |
| | 6 | 12 | .52±.27 | 4 | 0 | gBgD | O | IM |
| | 8 | 10 | .00±.27 | 5 | 10 | gBgD | nor-MDP | IM |
| Vaccination #2 | 1 | 11 | .01±.46 | - | - | O | O | O |
| | 4 | 12 | .98±.27 | - | - | gBgD | CFA | FP |
| | 6 | 12 | .52±.27 | 4 | O | gBgD | O | IM |
| | 8 | 8 | .01±.48 | 7 | 8 | gBgD | nor-MDP | IM |

| | | | | | | | | |
|---|---|---|---|---|---|---|---|---|
| ^{a} Temperature day 1 following vaccine - day 0 for vaccine (mean ± SD) (i.e., D22-21 or D 43-42) | | | | | | | | |
| ^{b} ND - not done | | | | | | | | |
| ^{c} Not applicable | | | | | | | | |
| ^{d} All animals had R let naired prior to vaccination except these | | | | | | | | |

The results in Table 7 also show that the relative efficacy of treatment increases as the interval between the initiation of immunotherapy and the onset of acute disease decreases. In animals which had received gBgD vaccine containing CFA beginning 8, 15, or 21 days after the initial infection, those animals which had received the vaccine the shortest time after intravaginal inoculation suffered the smallest number of lesion days, had the lowest percent of severe recurrences, and the fewest total episodes compared to the untreated control. The values obtained for the animals vaccinated 15 days after infection was higher than those obtained for the 8 day vaccination group, and the 21 day group was higher than the 15 day group. This effect is also shown in Figure 13, which presents a graph of the number of recurrences on the days after intravaginal inoculation for animals which were initially vaccinated 8, 15, or 21 days after the HSV-2 inoculation.

The data in Figure 13 was used to calculate the percent reduction in the rates of recurrent disease (See Section 6.1 for an explanation of the significance of the rate of recurrent disease). This data is presented in Table 11, where it may be seen that at earliest time periods, i.e., 14-50 days, the greatest percent reduction in the rate of recurrent disease was obtained by giving the initial vaccination at 8 days. However, from 51-92 days, the most effective protection was obtained by giving the initial vaccination 15 days after the intravaginal inoculation with HSV. The least protection occurred when the initial vaccination was given 21 days after the initial exposure to HSV-2.

It should be noted that in guinea pigs, an acute phase of the disease occurs during 14-21 days after viral infection; in this acute phase HSV induced lesions are found on the body of the animal. Therefore, the data in Table 13 show that the most effective protection at 51-92 days was obtained by administration of the vaccine during the acute phase of infection.

**Table 9**

| (Weekly Rate) Rates of Recurrence After Glycoprotein Treatment | | | | |
|---|---|---|---|---|
| Day 8 | 14-29 | 30-50 | 51-71 | 72-92 |
| UNRx | 3.30 | 2.48 | 1.42 | 1.15 |
| gBgD | 1.75 | 0.80 | 1.0 | 0.94 |
| % Control | 53.0% | 32.3% | 70.4 | 81.7 |
| % Reduction | 47.0% | 67.7% | 29.6% | 18.3% |
| Day 15 | 16-36 | 37-57 | 58-78 | 79-85 |
| UNRx | 3.27 | 2.09 | 1.42 | 0.97 |
| gBgD | 2.03 | 1.04 | 0.81 | 0.64 |
| % Control | 62.0% | 49.8% | 57.0% | 66.0% |
| % Reduction | 38.0% | 50.2% | 43.0% | 34.0% |
| Day 21 | 22-42 | 43-63 | 64-84 | 85-92 |
| UNRx | 2.82 | 2.18 | 1.03 | 0.85 |
| gBgD | 2.09 | 1.46 | 0.76 | 1.33 |
| % Control | 74.1% | 67.0% | 73.8% | 156.5% |
| % Reduction | 25.9% | 33.0% | 26.2% | 56.5% |

According to the present invention, vaccines are provided which are effective for therapeutic treatment of Herpes Simplex Virus Types 1 and 2 when administered post-viral infection.

## Claims

1. The use of
(a) an immunogenically active recombinant herpes simplex virus glycoprotein B (gB) polypeptide; and/or
(b) an immunogenically active recombinant herpes simplex virus glycoprotein D (gD) polypeptide;
wherein said polypeptide(s) is/are obtainable by recombinant technology, in making a vaccine for the post-infection treatment of Herpes Simplex Virus (HSV).

2. The use according to claim 1, wherein the gB is of type 1 or type 2, and/or wherein the gD is of type 1 or type 2.

3. The use according to claim 1 or claim 2, wherein the polypeptide is, or the polypeptides are, present in an amount effective to prevent recurrent HSV induced disease when the vaccine is administered to the individual during the acute stage of HSV infection.

4. The use according to any one of claims 1 to 3, wherein the vaccine includes a pharmacologically acceptable carrier and/or an adjuvant.

5. The use according to claim 4, wherein the adjuvant is N-acetyl-muramyl-L-alanyl-D-isoglutaminyl-L-alanine-2(1'-2'-dipalmitoyl-sn-glycero-3-hydroxyphosphoryloxy-ethylamine (MTP-PE).

6. The use according to claim 5 wherein the MTP-PE is in a low oil formulation.

7. The use according to any one of claims 1 to 6, wherein the individual infected with HSV is human.

## Patentansprüche

1. Verwendung
(a) eines rekombinanten immunogen wirksamen Glykoprotein B (gB)-Polypeptids vom Herpes simplex-Virus; und/oder
(b) eines rekombinanten immunogen wirksamen Glykoprotein D (gD)-Polypeptids vom Herpes simplex-Virus;
wobei das (die) Polypeptid(e) durch rekombinante Gentechnologie erhältlich ist (sind), zur Herstellung eines Impfstoffes für die post-infektiöse Behandlung des Herpes simplex-Virus (HSV).

2. Verwendung nach Anspruch 1, wobei gB vom Typ 1 oder Typ 2 ist, und/oder wobei gD vom Typ 1 oder Typ 2 ist.

3. Verwendung nach Anspruch 1 oder Anspruch 2, wobei das Polypeptid oder die Polypeptide in einer zur Verhinderung einer wiederkehrenden, durch HSV induzierten Krankheit wirksamen Menge vorhanden ist oder sind, wenn der Impfstoff dem Individuum während des akuten Stadiums der HSV-Infektion verabreicht wird.

4. Verwendung nach einem der Ansprüche 1 bis 3, wobei der Impfstoff einen pharmakologisch verträglichen Träger und/oder ein Adjuvans einschließt.

5. Verwendung nach Anspruch 4, wobei das Adjuvans N-Acetyl-muramyl-L-alanyl-D-isoglutaminyl-L-alanin-2-(1'-2'-dipalmitoyl-sn-glycero-3-hydroxyphosphoryloxy)-ethylamin (MTP-PE) ist.

6. Verwendung nach Anspruch 5, wobei MTP-PE in einer Niederöl-Formulierung vorliegt.

7. Verwendung nach einem der Ansprüche 1 bis 6, wobei das mit HSV infizierte Individuum ein menschliches Individuum ist.

## Revendications

1. Application de
(a) un polypeptide de glycoprotéine B (gB) du virus d'herpes simplex recombinant immunogéniquement actif; et/ou
(b) un polypeptide de glycoprotéine D (gD) du virus d'herpes simplex recombinant immunogéniquement actif; le(s)dit polypeptide(s)pouvant être obtenu(s) par une technique recombinante,
à la préparation d'un vaccin pour le traitement post-infectieux du virus de l'herpes simplex (HSV).

2. Application selon la revendication 1, dans laquelle la gB est de type 1 ou de type 2, et/ou dans laquelle la gD est de type 1 ou de type 2.

3. Application selon la revendication 1 ou la revendication 2, dans laquelle le polypeptide est, ou les polypeptides sont, présent(s) en une quantité efficace pour empêcher la récurrence d'une maladie induite par HSV lorsqu'on administre le vaccin à un individu au cours du stade aigu d'une infection par HSV.

4. Application selon l'une quelconque des revendications 1 à 3, dans laquelle le vaccin inclut un support pharmaceutiquement acceptable et/ou un adjuvant.

5. Application selon la revendication 4, dans laquelle l'adjuvant est la N-acétyl-muramyl-L-alanyl-D-isoglutaminyl-L-alanine-2(1',2'-dipalmitoyl-sn-glycéro-3-hydroxyphosphoryloxy-éthylamine (MTP-PE).

6. Application selon la revendication 5 dans laquelle la MTP-PE est une formulation à faible teneur en huile.

7. Application selon l'une quelconque des revendications 1 à 6, dans laquelle l'individu infecté par HSV est un être humain.
